# EUROPEAN PATENT APPLICATION

(11) **EP 2 053 052 A1**
(43) Date of publication of application: **29.04.2009**
(21) Application number: 07380289.4
(22) Date of filing: 23.10.2007
(51) Int. Cl.: C07D 513/04

(54) **Process for the preparation of 6-substituted imidazo[2,1-b]thiazole-5-sulfonyl halide**

(71) Applicant: Laboratorios del Dr. Esteve S.A., 08041 Barcelona (ES)
(72) Inventor: Mas-Prió, Josep, 08041 Barcelona (ES); Torrens-Jover, Antonio, 08041 Barcelona (ES)
(74) Representative: Carpintero Lopez, Francisco

(57) **Abstract**

The present invention is related to the process for the preparation of 6-substituted-imidazo[2,1-b]thiazole-5-sulfonyl halides of formula I 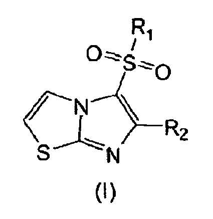 comprising gradually adding a compound of formula II 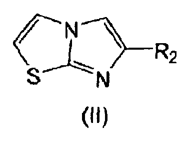 into a heated solution of R₁SO₃H
where R₁ represents F or Cl, and
R₂ represents H, F, Cl, Br, I, (C₁-C₆)alkyl, (C₁-C₆)alkoxy or trifluoromethyl.

## Description

### FIELD OF THE INVENTION

The present invention is related to the preparation of 6-substituted-imidazo [2,1-b] thiazole-5-sulfonyl halide of formula I 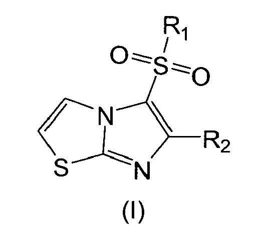 which are very useful and valuable intermediates in the synthesis of several compounds.

### BACKGROUND OF THE INVENTION

6-substituted-imidazo [2,1-b]thiazole-5-sulfonyl halides are very interesting intermediates that find wide use. For example, 6-chloroimidazo [2,1-b]thiazole-5-sulfonyl chloride is used in various pharmaceuticals applications for the synthesis of compounds that have demonstrated activity as 5-Hydroxytryptamine-6 ligands such as described in US20040209867**,** WO2007028460**,** W02007054257**,** EP1676841**,** EP1632491**,** W02006015867**,** WO2005014589**,** WO2005014045**,** WO2005014000**,** WO2005013979**,** WO2005013978**,** WO2005013977**,** WO2005013976**,** WO2004098588**,** WO2006002125**,** US2005101596**,** W02005012311**,** US2005020596**,** US2005020575**,** US2005009819 **or** US2004192749**.**

6-substituted-imidazo [2,1-b]thiazole-5-sulfonyl halides are also useful in the synthesis of sulfonylurea compounds with imidazo [2,1-b]thiazole moieties showing potent herbicidal activity as described in J.Pesticide Sci. 18, 183-189 (1993**)** and EP0238070**.**

J.Pesticide Sci. 18, 183-189 (1993**)** discloses the preparation of 6-chloro imidazo [2,1-b]thiazole-5-sulfonyl chloride. The method used to introduce the sulfonyl moiety comprises two steps. The synthesis comprises the reaction of compound (II) 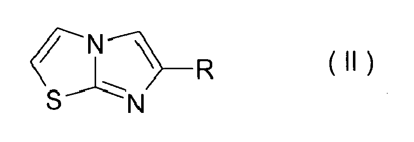 with chlorosulfonic acid in chloroform under reflux affording de sulfonic acids (III).

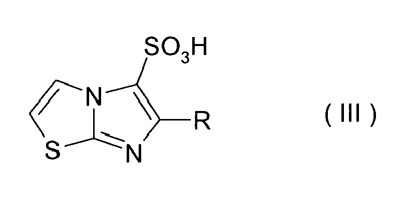

Reaction of sulfonic acids (III) with phosphorous oxychloride in the presence of triethylamine and temperature gives the corresponding sulfonylchlorides. In this procedure 6-chloroimidazo [2,1-b]thiazole-5-sulfonyl chloride was not isolated.

US 20040209867 discloses the same process as described in J.Pesticide Sci. 18, 183-189 (1993**)** which can be schematically represented as follows:

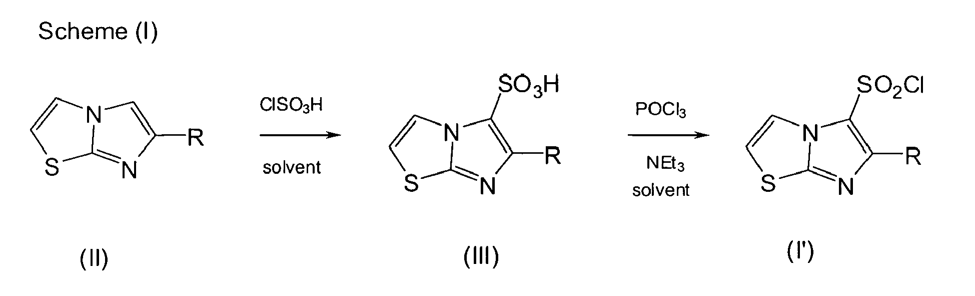

The present invention provides an alternative process to produce 6-substituted-imidazo [2,1-b] thiazole-5-sulfonyl halides which takes just one step. The process of the invention has the advantages of being a simpler, faster and highly efficient procedure, giving good yield and purity of 6-substituted-imidazo [2,1-b] thiazole-5-sulfonyl halides than the existing procedures. This makes the process of the present invention highly convenient for its implementation on an industrial scale.

### DESCRIPTION OF THE INVENTION

The present invention refers to a process for the preparation of a derivative of imidazo [2,1-b]thiazole-5-sulfonyl halide of formula I 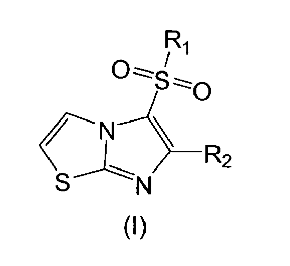 comprising gradually adding a compound of formula II 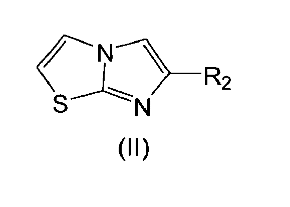 into a heated solution of R₁SO₃H
where R₁ represents F, Cl, Br or I and
R₂ represents H, F, Cl, Br, I, (C₁-C₆)alkyl, (C₁-C₆)alkoxy or trifluoromethyl.

The process in one step of the invention may be summarised as shown in Scheme II.

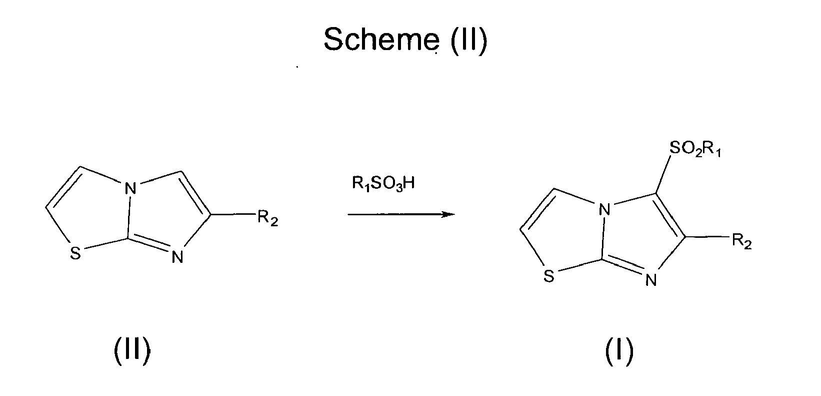

It is very important in the synthesis of derivatives of formula I that the halosulfonic acid (R₁SO₃H) is pre-heated before the compound of formula II is added. The addition of compound II in the heated solution of R₁SO₃H must be gradual because the reaction takes place with the release of an hydrogen halide such as HCl, HF, HBr or HI which might give rise to a violent reaction.

The temperature of the reaction mixture must be maintained during all the time the reaction is taking place (10 min-2 hs). Normally, the temperature at which the reaction is carried out must be between 60-140°C, preferably 100-130°C. It is also desirable that the halosulfonic acid is in excess (from 2.5 equivalents to 12 equivalents) so that substantially all 6-substituited imidazo [2,1-b]thiazole may be able to react.

The halosulfonic acid solution may be diluted, although, in a preferred embodiment of the invention the solution is just slightly diluted. In yet another preferred embodiment of the invention neat halosulfonic acid is used. The halosulfonic acid to be used would depend on the final derivative of imidazo [2,1-b]thiazole-5-sulfonyl halide that it is intended to be produced. For instance, if the final compound would be a 6-substituted-imidazo [2,1-b] thiazole-5-sulfonyl chloride, chlorosulfonic acid should be used as reactive. If on the contrary, 6-substituted-imidazo [2,1-b] thiazole-5-sulfonyl fluoride is sought, fluorosulfonic acid should be used instead of chlorosulfonic acid. In these sense, bromosulfonic acid and iodosulfonic acid may also be used to obtain the corresponding bromides and iodides respectively. Preparation of different 6-substituted-imidazo [2,1-b] thiazole-5-sulfonyl chlorides is specifically disclosed in examples 1 and 2.

In the course of the reaction, while the sulfonation takes place hydrogen halide is generated and so it is desirable to use a diluted sodium hydroxide solution to trapp this acid in excess. In a laboratory scale, a single hydroxide trapp may be used although for higher scales (industrial), it would be desirable to use a system composed of a first trapp with refrigerating water and then a second trap with the diluted hydroxide solution.

The yield according to this process is higher than 60% referred to the starting 6-substituted imidazo [2,1-b]thiazole. This crude material may be used directly without further purification.

In a particular embodiment of the invention, the reaction takes place between a compound of formula II where R₂ is Cl or Br and the halosulfonic acid is chlorosulfonic acid giving rise to either:
[1] 6-Chloroimidazo [2,1-b]thiazole-5-sulfonyl chloride, or
[2] 6-Bromoimidazo [2,1-b]thiazole-5-sulfonyl chloride.

The following examples are intended as means for illustrating the invention.

### Example 1: preparation of 6-Chloroimidazo [2,1-b]thiazole-5-sulfonyl chloride.

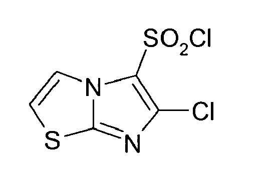

In a 1000 mL reaction flask, fitted with a mechanical stirrer, neat chlorosulfonic acid (3.60 mol, 240 mL) was placed and heated at 120 °C. (0.315 mol, 50 g) 6-chloroimidazo [2,1-b]thiazole was added gradually to the chlorosulfonic acid. The reaction mixture was stirred at 120 °C for 2 h. The hydrogen chloride generated during sulfonation was trapped using a diluted sodium hydroxide solution.

The syrupy liquid was quenched slowly, with mechanical stirring into ice (3.50 kg). The decomposition of the excess chlorosulfonic acid should be carried out in a hood and the efficient gas absorption trap was used. The solid 6-chloroimidazo [2,1-b]thiazole-5-sulfonyl chloride separates was collected by filtration, washed with water and dried under vacuum. Obtained 49.82 g (62 % yield) white solid. This crude material may be used directly.

IR (KBr) 3159, 3141, 1426, 1380, 1271, 1188, 1144, 1092, 732, 622, 562 cm⁻¹
1H NMR (300 MHz, DMSO-*d*₆) δ ppm 7,83 (d, *J*=4.5 Hz ,1 H) , 7,34 (d, *J*=4.5 Hz ,1 H).
MS (M+H)⁺ 173
M.P.: 140-142 °C

### Example 2: preparation of 6-Bromoimidazo [2,1-b]thiazole-5-sulfonyl chloride.

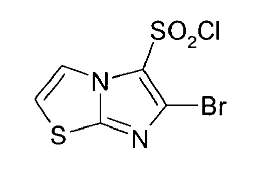

In a 25 mL flask, neat chlorosulfonic acid (30 mmol, 2.10 mL) was placed and heated at 120 °C. (2.5 mmol, 0.51 g) 6-bromoimidazo [2,1-b]thiazole was added gradually to the chlorosulfonic acid. The reaction mixture was stirred at 120 °C for 2 h. The hydrogen chloride generated during sulfonation was trapped using a diluted sodium hydroxide solution.

The syrupy liquid was quenched slowly, with stirring into ice (30 g). The decomposition of the excess chlorosulfonic acid should be carried out in a hood and the efficient gas absorption trap was used. The solid 6-bromoimidazo [2,1-b]thiazole-5-sulfonyl chloride separates was collected by filtration, washed with water and dried under vacuum. Obtained 0.47 g (61 % yield) white solid. This crude material may be used directly.

IR (KBr) 3153, 3135, 1415, 1380,1342, 1255, 1193, 1144, 1089, 727, 575 cm⁻¹
1 H NMR (300 MHz, DMSO-*d*₆) δ ppm 7,85 (d, *J*=4.5 Hz ,1H), 7,34 (d,
*J*=4.5 Hz ,1 H)
MS (M+H)⁺ 218
M.P.: 115-117 °C

## Claims

1. A process for the preparation of a derivative of imidazo [2,1-b]thiazole-5-sulfonyl halide of formula I 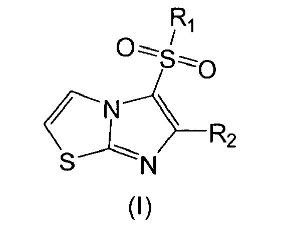 comprising gradually adding a compound of formula II 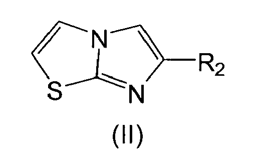 into a heated solution of R₁SO₃H
where R₁ represents F or Cl, and
R₂ represents H, F, Cl, Br, I, (C₁-C₆)alkyl, (C₁-C₆)alkoxy or trifluoromethyl.

2. A process for the preparation of a derivative of imidazo [2,1-b]thiazole-5-sulfonyl halide according to claim 1 where the solution of R₁SO₃H is a neat solution.

3. A process for the preparation of a derivative of imidazo [2,1-b]thiazole-5-sulfonyl halide according to claim 1 or 2 where the solution of R₁SO₃H is heated to a temperature between 60-140°C.

4. A process for the preparation of a derivative of imidazo [2,1-b]thiazole-5-sulfonyl halide according to claim 3 where the solution of R₁SO₃H is heated to a temperature of 100-130°C.

5. A process for the preparation of a derivative of imidazo [2,1-b]thiazole-5-sulfonyl halide according to any one of claims 1-4
where R₁ represents a F or Cl.

6. A process for the preparation of a derivative of imidazo [2,1-b]thiazole-5-sulfonyl halide according to any one of claims 1-4
where R₂ represents Cl or Br.

7. A process for the preparation of a derivative of imidazo [2,1-b]thiazole-5-sulfonyl halide according to claims 5 or 6 where the derivative prepared is one of:
[1] 6-Chloroimidazo [2,1-b]thiazole-5-sulfonyl chloride, or
[2] 6-Bromoimidazo [2,1-b]thiazole-5-sulfonyl chloride.
